# EUROPEAN PATENT APPLICATION

(11) **EP 1 801 561 A1**
(43) Date of publication of application: **27.06.2007**
(21) Application number: 05270102.6
(22) Date of filing: 22.12.2005
(51) Int. Cl.: G01N 21/05, G01N 21/35

(54) **Thermally Insulated Components**

(71) Applicant: Delphi Technologies, Inc., Troy, MI 48007 (US)
(72) Inventor: Kubis, Ruediger, 54329, Konz (DE)
(74) Representative: Jones, Keith William

(57) **Abstract**

A component (10) is formed by one or more bodies of shaped cellular glass to provide a self-supporting, thermally insulated component. The bodies may be shaped by machining from the solid and/or by fabrication. The component may be a gas analysis cell, the cellular glass providing a gas-tight volume.

## Description

This invention relates to thermally insulated components such as automotive components. The invention is particularly, but not exclusively, applicable to components which define a gas flow channel, most particularly to housings for optical gas sensors such as infrared absorption sensors.

There are many situations where it is desirable for a component which operates at an elevated temperature to be provided with thermal insulation. One such situation is in infrared absorption sensors used in real time on-line analysis of vehicle exhaust gases. The sensor typically comprises a detection cell or chamber formed of sheet metal, such as stainless steel, and having glass windows for passage of IR light from a source to a detector. The customary steel fabrication has a high thermal mass, leading to long start-up times until a stabile operating temperature of the detector can be maintained.

It is desirable for thermal stability for the cell to be thermally insulated; this is done by applying a thermally insulating material to the previously fabricated metal component. This increases the part count and assembly effort, and increases the volume occupied by the finished component. Also, commonly used insulating materials are not easy to handle and a robust and effective insulation is difficult to maintain over the desired lifetime of the component.

Cellular glass is known as a thermal insulation material, and is commercially available in the form of rectangular blocks and part-cylindrical shapes. Examples of commercially available cellular glass materials are FoamGlas® by Pittsburgh Corning and Cell-U-Foam^{™} by Cell-U-Foam Corp. Cellular glass has hitherto been used only as a thermally-insulating cladding applied over an existing structure.

The present invention provides a thermally insulated component in which the component itself is formed of cellular glass.

From another aspect, the invention provides a method of making a component, comprising forming the component from a body of cellular glass material.

Preferred features and advantages of the invention will be apparent from the following description and the claims.

An embodiment of the invention will now be described, by way of example only, with reference to the drawings, in which:

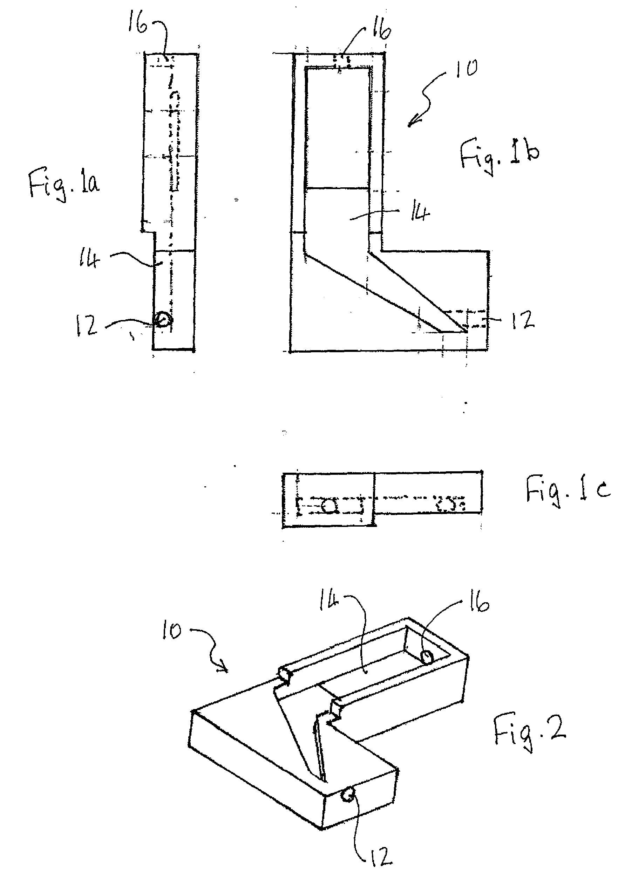

Figs. 1a, b and c are orthogonal views of a component forming the body of an IR absorption optical cell for exhaust gas monitoring; and
Fig. 2 is an isometric view of the same component.

Referring to the drawings, a component 10 forms a body portion for an optical gas analyser. The component 10 is shaped to provide a gas inlet 12, an open-topped gas passage 14, and a gas outlet 16. A cooperating cover member (not shown) is secured in use on top of the component 10 by a suitable adhesive and closes the gas passage 14. One or both of the component 10 and the cover member are provided with windows for transmission of infrared light through gas in the passage 14.

The component 10 (and preferably also the cover member) is fabricated from a block of cellular glass by machining, such as milling, drilling, routing and turning. The machined cellular glass component forms a self-supporting, gas-tight, thermally insulating product. It may be desirable for optically transparent windows to be located by metal fitments for accuracy, but such metal components will be small and easily fabricated.

Instead of machining, the component may be formed by other shaping methods; for example by cutting or grinding, or by fabrication from stock shapes of sheet, rod etc. by gluing or welding.

Thus, the invention allows a thermally insulated component to be built out of a minimum number of parts, with no further insulation material being required.

Although described principally in relation to automotive components, the invention may also be used in a range of other applications, such as medical devices, gas handling devices, and insulated storage.

Advantages of the use of cellular glass include:
- easy to machine
- very cheap (widely used for roof insulation)
- good insulating performance (thermal conductivity of 0.035-0.05 W/mºK)
- high robustness against thermal stress (working temperature of -260 to +430°C)
- low thermal mass, giving short light up/stabilisation time
- heating elements (e.g. electrical honeycomb heating elements) can be directly in contact with the housing without the risk of fire or melting of the material
- light weight (130-175 kg/m³)
- even parts with complex geometries can be realised with good precision
- gas tight
- water tight and non-absorptive
- not hygroscopic.

The material does suffer from being brittle, but if necessary this can be ameliorated by coating or injecting with a strengthening material such as a resin.

## Claims

1. A thermally insulated component in which the component itself is formed of cellular glass material.

2. A component according to claim 1, comprising one or more shaped bodies of cellular glass.

3. A component according to claim 2, comprising a plurality of shaped bodies secured together.

4. A component according to claim 3, in which said bodies are secured together by adhesive.

5. A component according to any of claims 2 to 4, in which said bodies are shaped by machining.

6. A component according to any preceding claim, in which the cellular glass is reinforced by coating or injection with a resin or polymer.

7. A component according to any preceding claim, in which the component comprises a flow channel for a gas.

8. A gas analyser cell comprising a component according to any preceding claim which forms a self-supporting, thermally insulated, cavity-defining member.

9. A method of making a component, comprising forming the component from a body of cellular glass material.

10. A method according to claim 9, in which the component is formed by securing together two or more shaped bodies of cellular glass.

11. A method according to claim 10, in which said bodies are shaped by machining.
